(19)

Europäisches Patentamt

European Patent Office

Office européen des brevets

(11)  **EP 3 584 796 A1**

(12)  **EUROPEAN PATENT APPLICATION**

(43) Date of publication:
**25.12.2019  Bulletin 2019/52**

(51) Int Cl.:
***G16H 10/20*** *(2018.01)*

(21) Application number: **18178532.0**

(22) Date of filing: **19.06.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **HENDRIKS, Monique**
**5656 AE Eindhoven (NL)**
• **DE PEE, Jente**
**5656 AE Eindhoven (NL)**
• **DADLANI MAHTANI, Pavankumar Murli**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54)  **DETERMINING MEDICAL TREATMENT PREFERENCES**

(57)  The invention discloses an apparatus (100) for determining a medical treatment preference of a subject. The apparatus (100) comprises a processor (102) configured to obtain clinical data relating to the subject; determine, based on the clinical data, a set of options available to the subject, wherein each option in the set of options is related to a medical treatment; generate, based on the set of options, a first question to be presented to the subject, wherein the first question is to elicit an indication of a preference of the subject regarding at least a first option of the set of options; train a predictive model based on an answer provided by the subject to the first question; and generate, based on the set of options and on an output of the predictive model, a second question to be presented to the subject, wherein the second question is to elicit an indication of a preference of the subject regarding at least a second option of the set of options.

Fig. 1

**Description**

FIELD OF THE INVENTION

The invention relates to determining a medical treatment preference of a subject. More particularly, the invention relates to determining a subject's medical treatment preferences using a series of specifically generated questions.

BACKGROUND OF THE INVENTION

Patient empowerment is a process through which people are able to gain greater control over decisions and actions affecting their health. Whereas, traditionally, a medical health professional may have made many important decisions regarding a patient's health, a rise in the need for patient empowerment has led to more decisions being made by patients themselves.

Patient empowerment has become important as a driver for lowering the cost of health care while at the same time improving the medical outcome for patients. As more alternative treatment options and treatment combinations become available, the choice regarding medical treatment is not only determined by the disease, but also by the patient's preferences. Furthermore, an increase in the number of people suffering from chronic diseases requires the management of diseases and symptoms to be more reliant on the patient than on the doctor.

Part of patient empowerment requires involvement of the patient in the decision making with respect to treatment. The best treatment option with respect to the patient's quality of life is largely dependent on the patient's personal values and preferences. For example, in the treatment of prostate cancer, some treatment options may lead to erectile dysfunction. Older prostate cancer patients and/or patients who are already experiencing erectile dysfunction might not be impacted very much, while younger patients might instead consider an alternative treatment option. In another example, adjuvant chemotherapy treatment (i.e. the use of chemotherapy in addition to another form of treatment) in breast cancer patients may reduce the risk of recurrence to some extent, but may also severely impact the patient's health in the short term and possibly also the long term. Thus, the patient's personal circumstances have a significant effect on the treatment they choose.

In order to involve the patient in decision making, it should be clear to the patient what the risks and consequences of different treatment options are and how they reflect on certain personal preferences and values of the patient besides the effectiveness of the treatment in curing the disease. It can, however, be difficult for a patient to express their views and values clearly.

One way of eliciting preferences of people regarding particular scenarios is through the use of discrete choice experiments (DCEs), which are intended to model human decision processes. In a typical discrete choice experiment, a set of questions is designed, in which two or more scenarios are presented and the participant or participants are asked to choose the best option for them. A number of participants is recruited (to resemble a population sample) and the questions are posed to these participants. Their answers are gathered and used in a model fitting procedure, which allows the researcher to draw general conclusions about preferences for a population having a certain sets of attributes. By obtaining responses from a large number of respondents, a reasonable understanding of the preferences of the population of respondents can be gained using relatively few questions. However, if a discrete choice experiment is performed using just one respondent, then a prohibitively large number of questions would need to be asked to the respondent in order to gain a full understanding of their preferences.

Thus, while discrete choice experiments are useful for gaining a general understanding for a population of people, there are limits to the usefulness of discrete choice experiments when determining preferences of a single patient with regard to medical treatment options.

SUMMARY OF THE INVENTION

It would be useful to have a mechanism by which a subject's personal values and views can be understood. It would be particularly useful to be able to obtain and/or understand a subject's views regarding medical treatment options.

According to a first aspect, the invention provides an apparatus for determining a medical treatment preference of a subject, the apparatus comprising a processor configured to: obtain clinical data relating to the subject; determine, based on the clinical data, a set of options available to the subject, wherein each option in the set of options is related to a medical treatment; generate, based on the set of options, a first question to be presented to the subject, wherein the first question is to elicit an indication of a preference of the subject regarding at least a first option of the set of options; train a predictive model based on an answer provided by the subject to the first question; and generate, based on the set of options and on an output of the predictive model, a second question to be presented to the subject, wherein the second question is to elicit an indication of a preference of the subject regarding at least a second option of the set of options.

By basing each subsequent question on the outcome of the model following the subject's answer to the previous question, it is possible to gain a thorough understanding of the subject's preferences without having to ask a large number of questions to the subject.

In some embodiments, the processor may be further configured to train the predictive model based on an answer provided by the subject to the second question; and iteratively train the predictive model based on subsequent answers provided by the subject in response to generated questions. The predictive model may be iteratively trained until a defined criterion is met. The defined criterion may comprises at least one of a) an output of the predictive model has converged to within a threshold range; and b) a defined threshold number of questions has been generated.

The predictive model may, in some embodiments, comprise a multinomial logistic regression model.

In some embodiments, the processor may be configured to generate the first question and/or generate the second question based at least partially on the clinical data associated with the subject.

The processor may further be configured to determine, based on an output of the predictive model, an indication of a medical treatment preference of the subject.

The processor may, in some embodiments, be configured to provide an indication to the subject of an effect on the predictive model resulting from the answer provided by the subject to the first question.

In some embodiments, each option in the set of options may be representative of a consequence and/or a side-effect associated with a form of medical treatment.

The first question and/or the second question may, in some embodiments, comprise an indication of a risk associated with a consequence and/or side-effect. In some embodiments, the first question and/or the second question may comprise an indication of a severity associated with a consequence and/or side-effect.

The apparatus may further comprise a user interface for receiving an input from a user. In some embodiments, the apparatus may comprise a memory.

According to a second aspect, the invention provides a method for determining a medical treatment preference of a subject, the method comprising: obtaining clinical data relating to the subject; determining, based on the clinical data, a set of options available to the subject, wherein each option in the set of options is related to a medical treatment; generating, based on the set of options, a first question to be presented to the subject, wherein the first question is to elicit an indication of a preference of the subject regarding at least a first option of the set of options; training a predictive model based on an answer provided by the subject to the first question; and generating, based on the set of options and on an output of the predictive model, a second question to be presented to the subject, wherein the second question is to elicit an indication of a preference of the subject regarding at least a second option of the set of options.

In some embodiments, the method may further comprise training the predictive model based on an answer provided by the subject to the second question; and iteratively training the predictive model based on subsequent answers provided by the subject in response to generated questions. The predictive model may be iteratively trained until a defined criterion is met.

The defined criterion may, in some embodiments, comprise at least one of a) an output of the predictive model has converged to within a threshold range; and b) a defined threshold number of questions has been generated.

According to a third aspect, the invention provides a computer program product comprising a non-transitory computer readable medium, the computer readable medium having computer readable code embodied therein, the computer readable code being configured such that, on execution by a suitable computer or processor, the computer or processor is caused to perform any of the methods disclosed herein.

These and other aspects of the invention will be apparent from and elucidated with reference to the embodiments described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a simplified schematic of an example of an apparatus for determining a medical treatment preference of a subject;
Fig. 2 is a flowchart of an example of a method for determining a medical treatment preference of a subject;
Fig. 3 is a flowchart of a further example of a method for determining a medical treatment preference of a subject;
Fig. 4 is a flowchart of a further example of a method for determining a medical treatment preference subject; and
Fig. 5 is a simplified schematic of a computer-readable medium in communication with a processor.

DETAILED DESCRIPTION OF EMBODIMENTS

Embodiments disclosed herein enable a user to gain understanding of a subject's views, values and/or preferences regarding medical treatment options available to the subject. Discrete choice experiments are particularly useful for obtaining preferences of a population of people, but have limitations when preferences of individuals are required. The inventors of the present invention have recognised that discrete choice experiment-type questioning may be used to understand the preferences of an individual if the line of questioning is selected in an intelligent manner. For example, according to embodiments disclosed herein, a question presented to a subject may be generated based on the subject's response to a previous question or a previous set of questions.

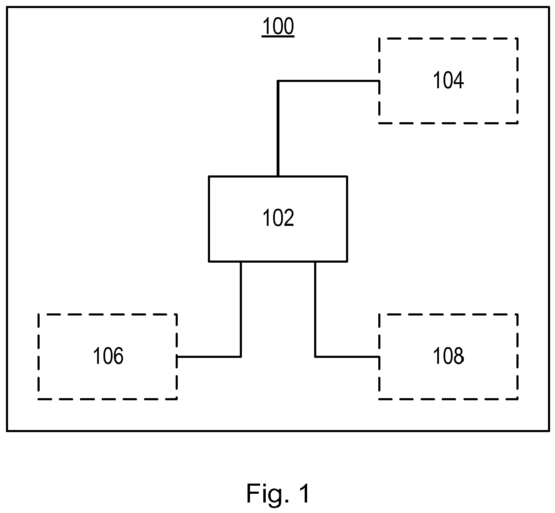

Fig. 1 shows a block diagram of an apparatus 100 that can be used for determining a medical treatment preference of a subject. With reference to Fig. 1, the apparatus 100 comprises a processor 102 that controls the operation of the apparatus 100 and that can implement the methods described herein. The apparatus 100 may further comprise a memory 106 comprising instruction data representing a set of instructions. The memory 106 may be configured to store the instruction data in the form of program code that can be executed by the processor 102 to perform the method described herein. In some implementations, the instruction data can comprise a plurality of software and/or hardware modules that are each configured to perform, or are for performing, individual or multiple steps of the method described herein. In some embodiments, the memory 106 may be part of a device that also comprises one or more other components of the apparatus 100 (for example, the processor 102 and/or one or more other components of the apparatus 100). In alternative embodiments, the memory 106 may be part of a separate device to the other components of the apparatus 100. For example, the apparatus 100 may be implemented as part of a cloud computing environment.

The processor 102 of the apparatus 100 can be configured to communicate with the memory 106 to execute the set of instructions. The set of instructions, when executed by the processor may cause the processor to perform steps of the methods described herein. The processor 102 can comprise one or more processors, processing units, multi-core processors or modules that are configured or programmed to control the apparatus 100 in the manner described herein. In some implementations, for example, the processor 102 may comprise a plurality of processors, processing units, multi-core processors and/or modules configured for distributed processing. It will be appreciated by a person skilled in the art that such processors, processing units, multi-core processors and/or modules may be located in different locations and may each perform different steps and/or different parts of a single step of the methods described herein.

In some embodiments, as illustrated in Fig. 1, the apparatus 100 may comprise at least one user interface 104 configured to receive any of the user inputs described herein. The user interface 104 may allow a user of the apparatus 100 to manually enter instructions, data, or information relating to the method described herein. For example, a user interface 104 may be used by a user (e.g. a subject) to provide a response to a question. In some embodiments, the user interface 104 may be used to present a question to the subject. In other embodiments, the user interface 104 may be used by a user (e.g. a medical professional) to input data relating to the subject. The user interface 104 may be any type of user interface that enables a user of the apparatus 100 to provide a user input, interact with and/or control the apparatus 100. For example, the user interface 104 may comprise one or more switches, one or more buttons, a keypad, a keyboard, a mouse, a touch screen, a microphone or an application (for example, on a tablet or smartphone), or any other user interface, or combination of user interfaces that enables the user to provide data to the apparatus.

In some embodiments, the user interface 104 (or another user interface of the apparatus 100) may enable rendering (or output or display) of information, data or signals to a user of the apparatus 100. As such, a user interface 104 may be for use in providing a user of the apparatus 100 (for example, a medical professional, a healthcare provider, a healthcare specialist, a care giver, a subject, or any other user) with information relating to or resulting from the method according to embodiments herein. The processor 102 may be configured to control one or more user interfaces 104 to provide information resulting from the method according to embodiments described herein. For example, the processor 102 may be configured to control one or more user interfaces 104 to render (or output or display) of data (e.g. clinical data) using the methods described herein, questions generated as a result of the methods described herein and/or any other outputs of the methods described herein, such as an indication of the subject's preference or preferences regarding one or more medical treatment options. The user interface 104 may, in some embodiments, comprise a display screen, a graphical user interface (GUI) or other visual rendering component, one or more speakers, one or more microphones or any other audio component, one or more lights, a component for providing tactile feedback (e.g. a vibration function), or any other user interface, or combination of user interfaces for providing information relating to, or resulting from the method, to the user. In some embodiments, the user interface 104 may be part of a device that also comprises one or more other components of the apparatus 100 (for example, the processor 102, the memory 106 and/or one or more other components of the apparatus 100). In alternative embodiments, the user interface 104 may be part of a separate device to the other components of the apparatus 100.

In some embodiments, as illustrated in Fig. 1, the apparatus 100 may also comprise a communications interface (or circuitry) 108 for enabling the apparatus 100 to communicate with any interfaces, memories and devices that are internal or external to the apparatus 100. The communications interface 108 may communicate with any interfaces, memories and devices wirelessly or via a wired connection.

It will be appreciated that Fig. 1 shows the components required to illustrate this aspect of the disclosure and, in a practical implementation, the apparatus 100 may comprise other components in addition to those shown. For example, the apparatus 100 may comprise a battery or other power supply for powering the apparatus 100 or means for connecting the apparatus 100 to a mains power supply.

As noted above, the processor 102 is configured to perform steps of the methods described herein. In some embodiments, a memory (e.g. the memory 106) may be configured to store a set of instructions which, when executed by the processor 102 of the apparatus 100, cause the processor 102 to perform steps or functions as discussed below. The processor 102 is configured to obtain clinical data relating to the subject. The processor 102 may be further configured to determine, based on the clinical data, a set of options available to the subject, wherein each option in the set of options is related to a medical treatment. The processor 102 may be further configured to generate, based on the set of options, a first question to be presented to the subject, wherein the first question is to elicit an indication of a preference of the subject regarding at least a first option of the set of options. The processor 102 may be further configured to train a predictive model based on an answer provided by the subject to the first question. The processor 102 may be further configured to generate, based on the set of options and on an output of the predictive model, a second question to be presented to the subject, wherein the second question is to elicit an indication of a preference of the subject regarding at least a second option of the set of options.

Thus, each subsequent question presented to the subject may be based on an answer provided by the subject to a previously-asked question. In this way, questions may be generated in order to elicit the appropriate information from the subject needed to obtain a fully understanding of the subject's preferences. By generating specific questions in this way, the total number of questions that need to be presented to the subject can be reduced, compared to a single, static questionnaire that is to be presented to a group of subjects. Thus, a sufficient understanding of an individual subject can be obtained using a relatively small number of questions.

The various functions performed by the processor 102 are discussed in greater detail below.

The clinical data relating to the subject may be obtained, for example, from a database containing clinical data of one or more subjects. The clinical data may, for example, be obtained from a health record (e.g. an electronic health record) associated with the subject. Clinical data and/or health records may be stored and/or maintained by a medical facility treating the subject, or in a central storage facility, such as a picture archiving and communication system (PACS). The clinical data may include characteristics of the subject such as an age of the subject, a gender of the subject, a medical history of the subject, details of any comorbidities associated with the subject, details of any past or current symptoms experienced by the subject, and the like. The clinical data may also include details relating to family members of the subject, such as a family history of any relevant medical issues. In some embodiments, the clinical data may include details or characteristics of a condition or disease to be treated. For example, the clinical data may include an indication of a clinical tumour stage given in respect of a tumour.

The clinical data may be obtained by the processor from a storage medium (e.g. electronic health record) automatically. In other embodiments, clinical data may be entered manually by a user (e.g. a medical professional, such as a radiologist), for example via the user interface 104.

Once the processor 102 has obtained the clinical data, it uses the clinical data to determine a set of options available to the subject, which relate to a medical treatment. In some embodiments, the options may relate to different treatments available to the subject, for example to treat a medical condition from which the subject is suffering. The options available to the subject depend on information included in the obtained clinical data; for example the clinical data may include details of one or more diseases from which the subject is suffering, and the options (e.g. treatment options) may relate to those diseases. Similarly, treatment options available to a subject may depend on the characteristics of the subject, such as the subject's gender or age. Thus, the determined treatment options may include only those treatment options appropriate for the subject based on their characteristics. Other information included in the clinical data may be used to determine the set of options available to the subject. In other embodiments, each option of the set of options may relate to a side-effect associated with a particular treatment option. For example, for one treatment option for a particular medical condition, there may be a very significant risk of a particular side-effect which, given the subject's situation (e.g. the subject's age, comorbidities and the like) is of relatively little concern to the subject. However, for another treatment option for the same medical condition, there may be a relatively small risk of a different side-effect which would be particularly undesirable for the subject. Thus, it is helpful to gain the subject's views on the different options available.

The options may be referred to as attributes. Thus, the processor 102 may be considered to determine a set of attributes available to the subject, each attribute relating to a medical treatment. In some examples, each attribute may comprise a side-effect associated with a medical treatment option. In some examples, an attribute may comprise a particular measure or characteristic; for example, an attribute may comprise a risk associated with a side-effect (e.g. where 0% represents no risk of suffering from the side-effect and 100% represents certainty that the subject will suffer from the side-effect) or a severity associated with the side-effect. In some examples, the severity of a side-effect may be defined on a scale of 1 to 5, with 1 representing a low severity (e.g. a urinary dysfunction leading to increased bathroom use) and 5 representing a high severity (e.g. a urinary dysfunction requiring the use of adult diapers for the rest of the subject's life).

It can be difficult for a subject to decide between two different options (e.g. two different side-effects associated with different treatment options, particularly if the subject does not have all of the relevant information (e.g. risk, severity, and so on) associated with each option available. Therefore, as discussed below, the present invention formulates questions to be presented to the subject which enable the subject to offer an informed view regarding the different options, so that a decision may be made as to which treatment option is most appropriate for the subject.

Each attribute/option may have one or more associated levels. For example for a particular side-effect, an attribute of 'risk' may have levels defined in terms of the "best-case risk", the "average case risk" and the "worst-case risk". More or fewer levels may be defined for each attribute. In the example shown in Tables 1 and 2 below, three possible treatment options are considered (i.e. Treatment A, Treatment B and Treatment C), and each treatment option has three possible side-effects (i.e. SF1, SF2 and SF3). The side-effects for the different treatment options may be the same or different. In addition to the side-effects SF1, SF2 and SF3, a list of other consequences is given for each treatment option. For each side-effect, two attributes are given: risk and severity. For each attribute, two different levels are described. In this example, a level representing an average case scenario is selected based on the mean chance of the side-effect occurring, and a level representing the worst-case scenario is chosen to be one standard deviation ("st-dev") above the mean. In other examples, the levels may be defined differently. For example, the level representing the worst-case scenario could be defined as being two standard deviations above the mean. Similarly, as noted above, a level representing the best-case scenario could also be included.

Table 1: Example of three treatment options, each having three possible side-effects.

| Side-effect | Treatment A | | | | | Treatment B | | | | | Treatment C | | | |
|---|---|---|---|---|---|---|---|---|---|---|---|---|---|---|
| SF1 | | | mean | st-dev | | | | mean | st-dev | | | | mean | st-dev |
| | | Risk | 0.3 | 0.5 | | | Risk | 0.2 | 0.01 | | | Risk | 0.7 | 0.1 |
| | | Severity | 2 | 1 | | | Severity | 4 | 1 | | | Severity | 2 | 1 |
| SF2 | | | mean | st-dev | | | | mean | st-dev | | | | mean | st-dev |
| | | Risk | 0.3 | 0.05 | | | Risk | 0.1 | 0.1 | | | Risk | 0.5 | 0.3 |
| | | Severity | 3 | 0.2 | | | Severity | 3 | 1 | | | Severity | 3 | 2 |
| SF3 | | | mean | st-dev | | | | mean | st-dev | | | | mean | st-dev |
| | | Risk | 0.6 | 0.2 | | | Risk | 0.5 | 0.1 | | | Risk | 0.5 | 0.1 |
| | | Severity | 1 | 1 | | | Severity | 1 | 1 | | | Severity | 2 | 1 |
| Other | Overnight hospital stay. 14 day recovery period. | | | | | Treatment duration: 6 months, weekly visits to the hospital. | | | | | Monthly check-ups over a period of 1 year. | | | |

The numerical values of the mean and standard deviation defined in Table 1 may be determined by a medical professional, or may be based on known or historical data relating to previous cases. Based on the numerical values in Table 1, a set of attributes and associated levels may be defined for each side-effect associated with each treatment option, as shown for example in Table 2.

Table 2: Attributes and levels for each treatment option, based on the example of Table 1.

|  | Attribute |  | Level |
| --- | --- | --- | --- |
| **Treatment A** | SF1 | Risk | Average case: 30% risk of developing the side-effect |
|  |  |  | Worst case: 80% risk of developing the side-effect |
|  |  | Severity | Average case: severity level 1 |
|  |  |  | Worst case: severity level 3 |
|  | SF2 | Risk | Average case: 30% risk of developing the side-effect |
|  |  |  | Worst case: 35% risk of developing the side-effect |
|  |  | Severity | Average case: severity level 3 |
|  |  |  | Worst case: severity level 3.2 |
|  | SF3 | Risk | Average case: 60% risk of developing the side-effect |
|  |  |  | Worst case: 80% risk of developing the side-effect |
|  |  | Severity | Average case: severity level 1 |
|  |  |  | Worst case: severity level 2 |
|  | Other |  | Overnight hospital stay. 14 day recovery period. |
| **Treatment B** | SF1 | Risk | Average case: 20% risk of developing the side-effect |
|  |  |  | Worst case: 21% risk of developing the side-effect |
|  |  | Severity | Average case: severity level 4 |
|  |  |  | Worst case: severity level 5 |
|  | SF2 | Risk | Average case: 10% risk of developing the side-effect |
|  |  |  | Worst case: 20% risk of developing the side-effect |
|  |  | Severity | Average case: severity level 3 |
|  |  |  | Worst case: severity level 4 |
|  | SF3 | Risk | Average case: 50% risk of developing the side-effect |
|  |  |  | Worst case: 60% risk of developing the side-effect |
|  |  | Severity | Average case: severity level 1 |
|  |  |  | Worst case: severity level 2 |
|  | Other |  | Treatment duration: 6 months, weekly visits to the hospital. |
| **Treatment C** | SF1 | Risk | Average case: 70% risk of developing the side-effect |
|  |  |  | Worst case: 80% risk of developing the side-effect |
|  |  | Severity | Average case: severity level 2 |
|  |  |  | Worst case: severity level 3 |
|  | SF2 | Risk | Average case50: % risk of developing the side-effect |
|  |  |  | Worst case: 80% risk of developing the side-effect |
|  |  | Severity | Average case: severity level 3 |
|  |  |  | Worst case: severity level 5 |

(continued)

|  | Attribute |  | Level |
| --- | --- | --- | --- |
|  | SF3 | Risk | Average case: 50 % risk of developing the side-effect |
|  |  |  | Worst case: 60% risk of developing the side-effect |
|  |  | Severity | Average case: severity level 2 |
|  |  |  | Worst case: severity level 3 |
|  | Other |  | Monthly check-ups over a period of 1 year. |

Based on the determined set of options, or attributes, the processor 102 generates a first question to be presented to the subject. In some embodiments, the first question may be generated at random based on the attributes/options. The first question is intended to elicit an indication of the subject's preference regarding at least one of the options in the determined set of options (e.g. the attributes shown in Table 2). In other words, in the example where each option relates to a side-effect associated with a treatment option, the first question is intended to elicit the subject's preference or views regarding the risk or severity of that particular side-effect. In some examples, a question may be generated that refers to two different options; one option relating to a first medical treatment and another option relating to a second medical treatment. For example, the question may refer to one side-effect associated with the first medical treatment and another side-effect associated with the second medical treatment. The subject's response to that question provides an indication of the subject's views on the two different side-effects and, based on that response, an initial view regarding which treatment option is most appropriate can be developed. Some questions, particularly the first question to be generated, may refer to more than two different options.

A single question to be answered by the subject is unlikely to be sufficient to gain a thorough understanding of the subject's views, particularly when multiple treatment options exist, and each treatment option has a number of associated side-effects. Therefore, multiple questions are presented to the subject.

The answer provided by the subject to the first question is used by the processor 102 to train a predictive model. The answer provided by the subject may be converted into a numerical format.

In some embodiments, the predictive model may comprise a multinomial logistic regression model. In other embodiments, other types of predictive model may be used. For example, the predictive model may comprise a multinomial logit model or a conditional logistic or logit model. A conditional logit model is able to model situations where a subject is indifferent towards two options (e.g. where a subject prefers option A in a set of options A, B and C, but has no preference with regard to B and C). A predictive model predicts, or provides a likelihood of, a particular outcome in view of an input and one or more parameters. In multinomial logistic regression, the probabilities of different possible outcomes can be calculated given a set of independent variables. Using multinomial logistic regression, the probability that a certain combination of levels is chosen over other combinations of levels (e.g. a subject prefers a low-risk but potentially high-severity side-effect over a high-risk but potentially low-severity side-effect) can be expressed generally by:

$$P_i = G\big(x_i, x_{j,j\neq i}, \beta\big) \qquad\qquad \text{Equation 1}$$

where $P_i$ is the probability alternative $i$ is preferred; $x_i$ is a vector of attributes of alternative $i$; $x_{j,j\neq i}$ is a vector of attributes of alternatives other than $i$; and $\beta$ is a set of parameters giving the effects of variables on the probabilities $P_i$ (i.e. the probabilities of the user choosing certain alternatives). The parameters forming the set of parameters, $\beta$, are estimated statistically, based on the answers provided by the subject to the questions. In the example discussed above, where various treatment options each have a number of side-effects, $\beta$ is initialised as a unity vector: $\beta = (1, 1, ..., 1)$ of length *treatments* • #*attributes* = 3 • 7 = 21. The number of attributes, #*attributes,* here includes the "Risk" and "Severity" for each side-effect (SF1, SF2 and SF3), and a value representing the "Other" side-effects.

Once an answer to the first generated question has been received (e.g. by an input from other subject via the user interface 104), the predictive model (e.g. a multinomial logistic regression model) is trained. Training (or fitting the answer to) the predictive model causes the parameters in the set of parameters, $\beta$, to be updated in view of the subject's response, thereby providing a new value for $\beta$, referred to as $\beta_{new}$. The previous value of $\beta$ may be referred to as $\beta_{previous}$. Thus, a change in $\beta$ resulting from an answer provided by the subject may be given by:

$$\delta = \beta_{new} - \beta_{previous} \qquad\qquad \text{Equation 2}$$

Therefore, a probability that a particular treatment-attribute combination will be selected for use in the next question is given by:

$$p = \frac{\delta}{\max(\delta)}$$

Equation 3

In Equation 3, the probability, $p$, is a vector, and it follows that, the larger the change in $\beta$ (i.e. the larger $\delta$ is), the higher the probability, $p$. Thus, the treatment-attribute combination that results in the largest shift in the vector $\beta$ has the largest probability of being included in the next question, because it is not clear from a large change in $\beta$ how to value that treatment-attribute combination. However, the treatment-attribute combination that results in the smallest shift in the vector $\beta$ has the lowest probability of being included in the next question, because the value of that treatment-attribute combination would be considered to have stabilized already.

The processor 102 uses the output from the predictive model (i.e. the probability, $p$) to generate a second question to be presented to the subject. The generation of the second question is also based on the set of options/attributes. An attribute to be included in the next (e.g. the second) question is chosen probabilistically, with attributes that cause the highest shift in $\beta$ having a higher probability of being included. Based on the probability, $p$, a new level of attribute is selected to be included in the second question. Thus, for each treatment, $t$: for each attribute, $a$: with a probability $p_{t,a}$ a new level of the attribute $a$ is selected.

The second question is presented to the subject, and the answer provided by the subject to the second question is used to train the predictive model again. A new set of parameters, $\beta_{new}$, is determined based on the answer to the first question and on the answer to the second question, and the change in $\beta$ resulting from the second answer is calculated. The newly-calculated probability, $p$, is calculated and used to generate a third question to be presented to the subject. The process of generating questions, obtaining answers, and using the subjects answers to train the predictive model may be repeated for a defined number of cycles, or until some defined criterion is met. Thus, with every iteration of training, the entire set of answers that has been given is used to train the predictive model and provide a new, updated set of parameters, $\beta$. The difference between each new $\beta$ the previously-determined $\beta$ is used to generate the next question to be presented to the subject.

The cycle of generating questions and training the predictive model based on answers provided by the subject to the generated questions may be considered to be an iterative process. Thus, in some embodiments, the processor 102 may be further configured to train the predictive model based on an answer provided by the subject to the second question; and iteratively train the predictive model based on subsequent answers provided by the subject in response to generated questions. The predictive model may be iteratively trained until a defined criterion is met. The defined criterion may be selected depending on the intention of the user. In some embodiments, the defined criterion may comprise at least one of: a) an output of the predictive model has converged to within a threshold range; and b) a defined threshold number of questions has been generated. In other words, in some examples, the predictive model may be iteratively trained until an output of the predictive model has converged to a desired level. Convergence of the output of the predictive model may indicate that asking further questions will not have a significant effect on the understanding of the subjects and preferences. Thus, once the output of the predictive model has converged to within the threshold range, it may be determined that sufficient knowledge of the subject's views and preferences has been obtained. In other examples, the predictive model may be itertively trained until the subject has answered a threshold number of questions. For example, iterative training of the predictive model may be stopped once all of the available questions have been generated and asked. Alternatively, an upper threshold of the number of questions to be answered by subject may be chosen in order to reduce question-answering fatigue of the subject.

Another way of reducing question-answering fatigue of the subject to whom the questions are presented is to provide the subject with some information to explain why a particular question is being asked. For example, the question may be presented with an explanation of why their response to the previous question has led to the generation of the current question. In some examples, a question may be presented with an explanation of what it is intended to be gained (e.g. the possible effect on the predictive model) from an answer to the question. Thus, the processor 102 may be configured to provide an indication to the subject of an effect on the predictive model resulting from the answer provided by the subject to the first question. In some examples, the processor 102 may be configured to provide an indication to the subject of an effect on the predictive model resulting from an answer provided by the subject to one or more of the questions (e.g. the second and subsequent questions). The information presented to the subject to explain the relevance of a question may, for example, include an indication of why attributes and/or levels have changed from one question to the next. In other words, the subject may be informed of their views or preferences as determined based on the output of the predictive model, in response to a question being answered.

As noted above, the set of options, also referred to as attributes, may relate to side-effects of medical treatments and, in some embodiments, the options/attributes may relate to particular characteristics of those side-effects, such as a risk of the side-effects occurring, or a severity of the side-effect. Thus, in some embodiments, each option in the set of options may be representative of a consequence and/or a side-effect associated with a form of medical treatment. A "consequence" may, for example, comprise a consequence or requirement listed in the "Other" row of Table 1. In other words, an option/attribute may include a repercussion or other requirement that may not necessarily be considered to be a medical side-effect. The first question and/or the second question may, in some embodiments, comprise an indication of a risk associated with a consequence and/or side-effect. In some embodiments, the first question and/or the second question may comprise an indication of a severity associated with a consequence and/or side-effect. In some embodiments, the first question and/or the second question may comprise an indication of both a risk and a severity associated with a consequence and/or side-effect.

According to some embodiments, the apparatus 100 may employ techniques to ensure that questions presented to the subject are relevant to the subject. This may help to maintain the subject's interest throughout the questioning, and may help to ensure that the subject understands the questions being asked. In some embodiments, the processor 102 may be configured to generate the first question and/or generate the second question based at least partially on the clinical data associated with the subject. In other words, the obtained clinical data may be used to ensure that the questions presented to the subject are relevant to the subject. For example, rather than asking a generic question relating to a side-effect associated with a particular medical treatment, the question may be generated that contains a realistic scenario for the subject, based on the subjects clinical data. For example, a question to be answered by a female subject should not refer to a side-effect, such as erectile dysfunction, that would only be relevant to a male subject.

Similarly, questions may be phrased using examples, analogies and/or scenarios that are more likely to be familiar to and/or understood by the subject. Thus, the phrasing of the questions may also be based on the obtained clinical data. For example, an indication of a risk associated with a particular side-effect may be presented to a subject in terms of an equivalent risk, such as the risk of being involved in the car accident or a plane crash, or the chance of winning the lottery, rather than as a percentage.

Once the iterative training of the predictive model has ended (for example upon meeting the defined criterion) the processor 102 may be configured to generate and/or provide an indication of a result of the training or an output of the predictive model. For example, in some embodiments, the processor 102 may be configured to determine, based on an output of the predictive model, an indication of a medical treatment preference of the subject. Such an indication may, for example, comprise an indication of the most appropriate treatment option for the subject given that subject's views on the possible side-effects associated with the available medical treatment options. Such an indication may be presented on a display screen (e.g. the user interface 104) or communicated to the subject and/or to a medical professional (e.g. via the communications interface 108).

The generation of questions and the fitting of answers to those questions to a predictive model (i.e. the training of the predictive model) may be generalised as shown below. Various "scenarios" relevant to the subject may be considered, and used to form the basis of questions. Each "scenario" relates to a different possible future state for the subject. Each medical treatment option has a number of possible future states, defined by the risk and severity of the side-effects associated with the treatment, and by other consequences associated with the treatment, such as hospitalisation, recovery time, and so on. In this generalisation, we assume that there are:

$n$ treatments $t_1 \dots t_n$;
$m$ side-effects $s_1 \dots s_m$;
$x$ attribute of each side-effect (e.g. risk and severity) $a_1 \dots a_x$; and
$y$ levels of each attribute (e.g. average case, worst-case, and so on) $a_{i,1} \dots a_{i,y}$ where $I \in 1 \dots x$.

A question to be generated by the processor 102 may include two scenarios, of which the subject is to choose which one they prefer. In some embodiments, a question may include more than two scenarios. A scenario may, therefore, consist of:
for a treatment t:
for each side-effect s:
some $a_{i,j}$

Each scenario is personalised in the choice for $a_{i,j}$ (e.g. the average case and the worst-case of the expected risk and severity for each side-effect) based on subject-specific parameters, such as tumour size, tumour location, the age of the subject, pre-existing medical conditions, and so on. Such parameters may, for example, be obtained from the clinical data relating to the subject.

The combination of $a_{i,j}$s to be used in the scenarios presented in the subsequent question is determined probabilistically, based on the parameter $\delta = (\delta_1 \dots \delta_{x \cdot y})$ (see Equation 2). The parameter $\delta$ is then used to calculate the probability vector, $p$, as discussed above.

Consider an example in which a subject is suffering from prostate cancer. For low-grade prostate cancer there are many available treatment options. For example, prostate cancer may be treated with radical prostatectomy (i.e.

surgery to remove the prostate), external beam radiation therapy or brachytherapy. These three treatment options all have different associated potential side-effects, including urinary dysfunction, bowel problems and impotence.

The risks and expected severity of the side-effects are different for each treatment, but also vary based on the characteristics of the subject. For example, the risks and severity of the side-effects may be influenced by the location of the tumour, the size of the tumour, the subject's age, and any pre-existing urinary, bowel or erectile problems. These example side-effects can be applied to the generalised notation above:

Table 3

| | |
|---|---|
| $n$ treatments $t_1 ... t_n$ | $t_1$ = radical prostatectomy, $t_2$ = external beam radiation, $t_3$ = brachytherapy |
| $m$ side-effects $s_1 ... s_m$ | $s_1$ = urinary dysfunction, $s_2$ = bowel problems, $s_3$ = impotence |
| $x$ attributes of each side-effect (e.g. risk and severity) $a_1 ... a_x$ | $a_1$ = risk of developing the side-effect after treatment, $a_2$ = expected severity of the side-effect |
| $y$ levels of each attribute (e.g. average case, worst-case, and so on) $a_{i,1} ... a_{i,y}$ where $l \in 1 ... x$ | $a_{1,1}$ = average risk of the side-effect for the individual patient, $a_{1,2}$ = worst-case scenario (i.e. average risk plus one standard deviation), $a_{2,1}$ = average expected severity of the side-effect for the individual patient, $a_{2,2}$ = worst-case scenario (i.e. average expected severity plus one standard deviation) |

Thus, an example of a question generated (e.g. by the processor 102) based on this example, assuming just two treatments are to be compared each question, might be:

*Which of the following options would you prefer?*

Table4

| Option 1 (for treatment radical prostatectomy) | Option 2 (for treatment external beam radiation) |
|---|---|
| In the worst case scenario, you have 80% risk of developing urinary dysfunction | In the worst case scenario, the severity of urinary problems would be grade 4 (wearing 5 or more pads per day) |
| On average, the severity level of Impotence would be grade 3 | The risk of impotence is 20% |
| Average risk of developing bowel problems is 10% | On average, grade of bowel problems would be 2 (loose stool) |

For the first question to be generated, the selection of which level and/or attribute to include in the question may be made at random. Thereafter, the selection of which level and/or attribute to include in a question may be based on the probability parameter, $p$. The probability, $p$, is a vector of length $x*y$, so has a value for each level of each attribute. Therefore, there is a probability $p$ for each side-effect. A question may be generated using the following logic:

for a treatment t:

for each side-effect s:

pick an $i,j$ combination at random from $(1 ...x*y)$ with probability distribution $p$;

present $a_{i,j}$ as the attribute-level combination for s for treatment t.

Based on the subject's response to a question, the predictive model may be trained in the following way. As noted above, the set of parameters, $\beta$ is initialised as a unity vector $(1 ... 1)$ of length $x*y$. The parameter $p$ may be initialised as:

$$p = \left( \frac{1}{x*y} ... \frac{1}{x*y} \right), \text{ a vector of length } x*y \qquad \text{Equation 4}$$

The predictive model is trained until:

$\Sigma_{1..x*y} |\beta_{new} - \beta_{previous}|$ is less than a defined threshold value, $\varepsilon$, or does not increase for z iterations.

Questions are presented to the subject, and responses subject are received, and the answers are used to train predictive model to obtain the set of parameters, $\beta$.

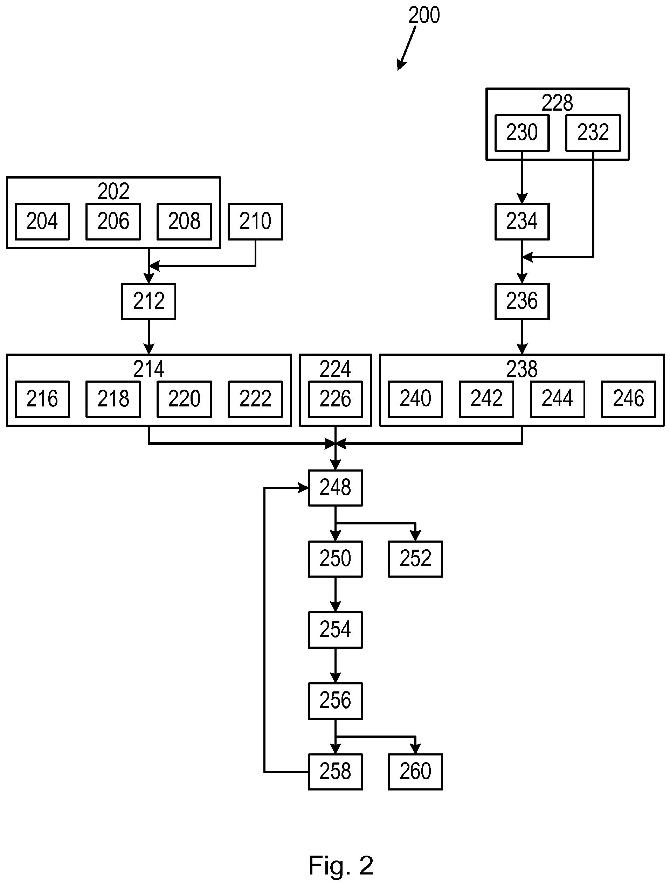

Embodiments of the invention will now be described with reference to specific examples and with reference to Figure 2. Figure 2 shows the flow of information when generating questions, and examples of the processes performed by the processor 102 when implementing the invention.

Block 202 represents data relating to a set of side-effects for a medical treatment option available to a subject. The data relating to the set of side-effects 202 may be obtained from the medical literature or from a knowledge database, for example, and may be personalised for the subject based on any available information relating to the subject. For example, the side-effects may be personalised based on the obtained clinical data relating to the subject. The data 202 may include a personalised side-effect risk profile 204 which may, for example, include various levels of risk (e.g. worst-case, best case, average case, and the like) described in terms of a mean risk and the standard deviation, as shown for example in Table 1 above. The data 202 may also include a personalised side-effect severity profile 206 which may, for example, include various levels of severity (e.g. worst-case, best case, average case, and the like) described in terms of the mean severity and a standard deviation, as shown for example in Table 1 above. The data 202 may also include descriptions 208 of each level of severity for the side-effects. Block 210 represents data relating to a set of risk profiles for well-known statistics (e.g. a risk of being involved in a car accident or plane crash, or the chance of winning the lottery, and so on). The data 210 may be used to phrase a question for the subject using an analogy that may be more familiar to them.

The data 202 and 210 may be provided to a description generator 212 which is capable of using known techniques to generate sentences and phrases to be used in questions, based on the data 202, 210. The description generator 212 outputs a set of descriptions 214 relating to the side-effects. The set of descriptions 214 may include a description or descriptions of an average side-effect risk 216, a description or descriptions of a "worst case" side-effect risk 218, a description of an average side-effect severity 220 and a description of a worst-case side-effect severity 222. In embodiments where additional levels of risk and severity are to be included, additional descriptions will be generated.

Block 224 represents data relating to other attributes, characteristics and/or consequences of the method of treatment. The data 224 may, for example, include a description 226 of consequences other than the side-effects are relevant to the treatment, such as treatment time, recovery time, the expected number of hospital visits, and the like. In some embodiments, the data 224 may include other relevant attributes of the treatment to be taken into account.

Block 228 represents data relating to expected survival rates and disease progression based on the medical treatment. The data 228 may be obtained from medical literature or from a knowledge database, for example, and may be personalised for the subject based on any available information relating to the subject. For example, the survival rate data and disease progression data may be personalised based on the obtained clinical data relating to the subject. The data 228 may, for example, include a personalised survival risk profile 230 which may, for example, include various levels of survival risk (e.g. worst-case, best-case, average case, and the like) described in terms of a mean survival risk and the standard deviation. The data may also include a personalised disease progression risk profile 232, which may also include various levels of disease progression risk (e.g. worst-case, best-case, average case, and the like) described in terms of a mean survival risk and the standard deviation. The personalised survival risk profile 230 may be used by a life expectancy calculator or simulator 234 to calculate a life expectancy of the subject having a particular medical condition, in view of factors relevant to the subject (e.g. based on their clinical data). An output from the life expectancy calculator 234 may be provided, along with the personalised disease progression risk data 232, to a description generator 236 capable of using known techniques to generate sentences and phrases to be used in questions, based on the data 230, 232. The description generator 236 outputs a set of descriptions 238 relating to the life expectancy and risk of disease progression. The set of descriptions 238 may include a description of an average life expectancy 240, a description of a worst-case life expectancy 242, a description of an average risk of progression to 244, and a description of a worst-case risk of progression 246.

The data 224 and the descriptions 214 and 238 are provided for use by a question generation algorithm 248. The question generation algorithm 248 may, for example, comprise an algorithm implemented using the processor 102. The question generation algorithm 248 may be considered to be adaptive or dynamic as each subsequent question generated is based on a response provided to the previous question or questions. The question generation algorithm 248 generates a question 250 to be presented to the subject. In some embodiments, as discussed above, the question generation algorithm 248 (or another module or function performed by the processor 102) may also generate an explanation 252 as to why the question is being asked, which may also be presented to the subject together with the question 250. The subject provides an answer 254 to the question 250, and the answer is used to train a predictive model (block 256). Training a predictive model may also be referred to as fitting the answer 250 to the model. The predictive model may provide as an output a subject-specific profile 258 describing the subject's preferences regarding various treatment options. Based on the output of the model, an explanation 260 may also be generated, which shows the subject how the output of the model has changed given their response to the last question asked.

If a defined criterion has been met (e.g. an output of the model has converged to within a threshold range, or a defined number of questions have been generated and/or presented to the subject), then the question generation process may be ended. Alternatively, if the defined criterion has not been met, then the process may return to block 248, where the question generation algorithm generates a further question.

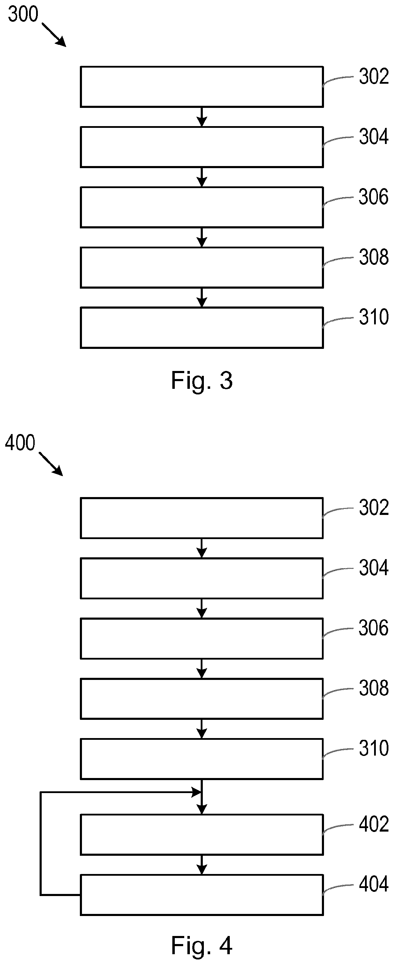

According to another aspect, a method for determining a medical treatment preference of the subject is provided. Fig. 3 is a flowchart of an example of such a method 300. The method comprises, at step 302, obtaining clinical data relating to the subject. At step 304, the method 300 comprises determining, based on the clinical data, a set of options available to the subject, wherein each option in the set of options is related to a medical treatment. The method 300 comprises, at step 306, generating, based on the set of options, a first question to be presented to the subject, wherein the first question is to elicit an indication of a preference of the subject regarding at least a first option of the set of options. At step 308, the method 300 comprises training a predictive model based on an answer provided by the subject to the first question. The method 300 comprises, at step 310, generating, based on the set of options and on an output of the predictive model, a second question to be presented to the subject, wherein the second question is to elicit an indication of a preference of the subject regarding at least a second option of the set of options. As noted above, the predictive model may comprise a multinomial logistic regression model.

The steps 302 to 310 of the method 300 may be considered equivalent to the functions performed by the processor 102, described above. Thus, in some embodiments, the method may be performed by a processor, such as the processor 102.

Fig. 4 is a flowchart of a further example of a method 400 for determining a medical treatment preference of the subject. The method 400 may comprise blocks of the method 300 discussed above. In some embodiments, the method 400 may further comprise, at step 402, training the predictive model based on an answer provided by the subject to the second question. At step 404, the method 400 may comprise iteratively training the predictive model based on subsequent answers provided by the subject in response to generated questions. The predictive model may be iteratively trained until a defined criterion is met. As discussed above, the defined criterion may be chosen depending on the intended purpose or outcome. In some embodiments, however, the defined criterion may comprise at least one of: a) an output of the predictive model has converged to within a threshold range; and b) a defined threshold number of questions has been generated.

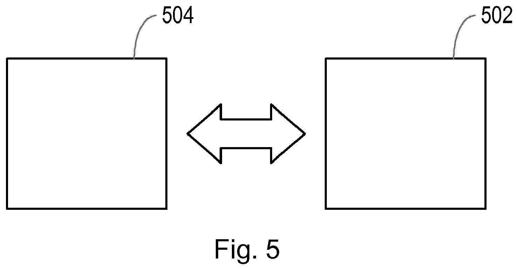

According to a further aspect, a computer program product is provided. Fig. 5 is a simplified schematic of a processor 502 connected to a computer-readable medium 504. According to some embodiments, a computer program product comprises a non-transitory computer-readable medium 504, the computer-readable medium having computer readable code embodied therein, the computer readable code being configured such that, on execution by a suitable computer or processor 502, the computer or processor is caused to perform the methods 300, 400 disclosed herein. The processor 502 may, in some embodiments, comprise or be similar to the processor 102 discussed above.

The processor 102, 502 can comprise one or more processors, processing units, multi-core processors or modules that are configured or programmed to control the apparatus 100 in the manner described herein. In particular implementations, the processor 102, 502 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or apparatus according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the apparatus and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1.  An apparatus (100) for determining a medical treatment preference of a subject, the apparatus comprising:
    a processor (102) configured to:

    obtain clinical data relating to the subject;
    determine, based on the clinical data, a set of options available to the subject, wherein each option in the set of options is related to a medical treatment;
    generate, based on the set of options, a first question to be presented to the subject, wherein the first question is to elicit an indication of a preference of the subject regarding at least a first option of the set of options;
    train a predictive model based on an answer provided by the subject to the first question; and
    generate, based on the set of options and on an output of the predictive model, a second question to be presented to the subject, wherein the second question is to elicit an indication of a preference of the subject regarding at least a second option of the set of options.

2.  An apparatus (100) according to claim 1, wherein the processor (102) is further configured to:

    train the predictive model based on an answer provided by the subject to the second question; and
    iteratively train the predictive model based on subsequent answers provided by the subject in response to generated questions;
    wherein the predictive model is iteratively trained until a defined criterion is met.

3.  An apparatus (100) according to claim 2, wherein the defined criterion comprises at least one of:

    a) an output of the predictive model has converged to within a threshold range; and
    b) a defined threshold number of questions has been generated.

4.  An apparatus (100) according to any of the preceding claims, wherein the predictive model comprises a multinomial logistic regression model.

5.  An apparatus (100) according to any of the preceding claims, wherein the processor (102) is configured to generate the first question and/or generate the second question based at least partially on the clinical data associated with the subject.

6.  An apparatus (100) according to any of the preceding claims, wherein the processor (102) is further configured to:
    determine, based on an output of the predictive model, an indication of a medical treatment preference of the subject.

7.  An apparatus (100) according to any of the preceding claims, wherein the processor (102) is configured to:
    provide an indication to the subject of an effect on the predictive model resulting from the answer provided by the subject to the first question.

8.  An apparatus (100) according to any of the preceding claims, wherein each option in the set of options is representative of a consequence and/or a side-effect associated with a form of medical treatment.

9. An apparatus (100) according to claim 8, wherein the first question and/or the second question comprises an indication of a risk associated with a consequence and/or side-effect.

10. An apparatus (100) according to claim 8 or claim 9, wherein the first question and/or the second question comprises an indication of a severity associated with a consequence and/or side-effect.

11. An apparatus (100) according to any of the preceding claims, further comprising:
a user interface for receiving an input from a user.

12. A method (300) for determining a medical treatment preference of a subject, the method comprising:

obtaining (302) clinical data relating to the subject;
determining (304), based on the clinical data, a set of options available to the subject, wherein each option in the set of options is related to a medical treatment;
generating (306), based on the set of options, a first question to be presented to the subject, wherein the first question is to elicit an indication of a preference of the subject regarding at least a first option of the set of options;
training (308) a predictive model based on an answer provided by the subject to the first question; and
generating (310), based on the set of options and on an output of the predictive model, a second question to be presented to the subject, wherein the second question is to elicit an indication of a preference of the subject regarding at least a second option of the set of options.

13. A method (300, 400) according to claim 11, further comprising:

training (402) the predictive model based on an answer provided by the subject to the second question; and
iteratively training (404) the predictive model based on subsequent answers provided by the subject in response to generated questions;
wherein the predictive model is iteratively trained until a defined criterion is met.

14. A method (300, 400) according to claim 12, wherein the defined criterion comprises at least one of:

a) an output of the predictive model has converged to within a threshold range; and
b) a defined threshold number of questions has been generated.

15. A computer program product comprising a non-transitory computer readable medium, the computer readable medium (504) having computer readable code embodied therein, the computer readable code being configured such that, on execution by a suitable computer or processor (502), the computer or processor is caused to perform the method of any of claims 12 to 14.

Fig. 1

200

228
230 232
234
236

202
204 206 208 210
212

214
216 218 220 222

224
226

238
240 242 244 246

248
250 252
254
256
258 260

Fig. 2

300

| 302 |
| 304 |
| 306 |
| 308 |
| 310 |

Fig. 3

400

| 302 |
| 304 |
| 306 |
| 308 |
| 310 |
| 402 |
| 404 |

Fig. 4

504 502

Fig. 5

Europäisches
Patentamt

European
Patent Office

Office européen
des brevets

# EUROPEAN SEARCH REPORT

Application Number

EP 18 17 8532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/072939 A1 (DAHAN ELIAV [US]) 13 March 2014 (2014-03-13) * paragraphs [0018] - [0097]; figure 2 * | 1-15 | INV. G16H10/20 |
| X | WO 2017/106770 A1 (COGNOA INC [US]; ABBAS HALIM [US]) 22 June 2017 (2017-06-22) * paragraphs [0186] - [0288] * | 1-15 | |
| A | Kirstin Early ET AL: "Dynamic Question Ordering: Obtaining Useful Information While Reducing User Burden", , 13 October 2016 (2016-10-13), XP055528039, DOI: 10.1184/R1/6716123 Retrieved from the Internet: URL:http://www.cs.cmu.edu/~kearly/files/proposal.pdf [retrieved on 2018-11-28] * the whole document * | 1-15 | |

TECHNICAL FIELDS SEARCHED (IPC)

G16H

The present search report has been drawn up for all claims

| Place of search | Date of completion of the search | Examiner |
|---|---|---|
| Munich | 29 November 2018 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 17 8532

This annex lists the patent family members relating to the patent documents cited in the above-mentioned European search report.
The members are as contained in the European Patent Office EDP file on
The European Patent Office is in no way liable for these particulars which are merely given for the purpose of information.

29-11-2018

| Patent document cited in search report | Publication date | Patent family member(s) | Publication date |
|---|---|---|---|
| US 2014072939 A1 | 13-03-2014 | NONE | |
| WO 2017106770 A1 | 22-06-2017 | CN 108780663 A | 09-11-2018 |
| | | EP 3394825 A1 | 31-10-2018 |
| | | WO 2017106770 A1 | 22-06-2017 |

EPO FORM P0459

For more details about this annex : see Official Journal of the European Patent Office, No. 12/82